# EUROPEAN PATENT APPLICATION

(11) **EP 4 321 150 A1**
(43) Date of publication of application: **14.02.2024**
(21) Application number: 22189337.3
(22) Date of filing: 08.08.2022
(51) Int. Cl.: A61K 8/9783, A61Q 7/00, A61K 36/18, A61P 17/14

(54) **COSMETIC TOPICAL COMPOSITION FOR IMPROVING CAPILLARY DENSITY CONTAINING AS ACTIVE INGREDIENT AT LEAST AN OILY EXTRACT OF MORINGA PEREGRINA SEEDS, METHOD FOR PREPARING THE COMPOSITION AND METHOD FOR COSMETIC TREATMENT OF HAIR**

(71) Applicant: AlUla Peregrina Trading Company, 43514 AlUla city (SA)
(72) Inventor: DODINET, Elisabeth, 12560 Saint Larent d'Olt (FR); BOURGETEAU, Vincent, 56130 Férel (FR)
(74) Representative: Loyer & Abello

(57) **Abstract**

The invention relates to the cosmetic field and more particularly to an oily extract of *Moringa peregrina* seeds as active ingredient for improving capillary density. The invention also relates to a method for preparing said composition and to cosmetic treatment of hair.

## Description

### Technical domain

The invention relates to the cosmetic field and more particularly to the field of active ingredients included in the formulation of skincare topical compositions. The invention refers more particularly to an oily extract of *Moringa peregrina* seeds as active ingredient for improving capillary density. The invention also relates to a method for preparing said composition and to cosmetic treatment of hair.

### Technical background

Those skilled in the art have known for a long time that natural hair loss in humans globally reflects the balance of the hair follicles between the alternating phases of growth (anagen) and the phases of loss (telogen). The average ratio of the number of follicles in the anagen phase to that in the telogen phase is around 9 (90/10). Natural hair loss is estimated at a few hundred hairs per day for a normal physiological state. It is also known that certain factors such as hormonal imbalance, physiological stress, malnutrition, can accentuate this phenomenon of hair loss. In certain dermatoses of the scalp with an inflammatory characteristic, such as, for example, psoriasis or seborrheic dermatitis, hair loss can be greatly accentuated. If we want to provide an effective response to the problem of hair loss, it is certainly important to stop the fall, but you also have to do everything you can to stimulate the growth of new hair.

Moreover, the risks of controversial ingredients in cosmetics have also been widely denounced. Chemical treatments are increasingly abandoned in favor of natural treatments. Natural treatments are very interesting for they are free of chemical substances that could harm both the skin and the hair.

In view of the above-cited constraints the Applicant has surprisingly discovered that a composition comprising, as active ingredient, at least one oily extract of *Moringa peregrina* seeds makes it possible to improve drastically the capillary density.

The Applicant has found that this composition was particularly effective for hair regrowth and/or for slowing down hair loss. It appears that the natural composition in itself provides improvement of capillary density without the need to add chemical ingredients.

The Moringaceae are a mono-generic family (only one genus, *Moringa adans*), an element of the Saharo-Sindian flora, constituted of between 12 and 14 species according to the authors, distributed from Eastern Africa to Asia. The genus is conventionally divided into three sections which are, however, not confirmed as monophyletic by the phylogenetic analyses. Said analyses have rather revealed clades centered on certain morphological characters: pachycauls ("bottle trees"); "tuberous trees" and those that are neither bottle trees nor tuberous trees ("slender trees"). The species *Moringa peregrina* (Forssk.) Fiori, belongs to the third group. The sparse genetic studies on the genus or the family confirm the reality of the species relative to the other species in the genus, notably with respect to Indian Moringa, *Moringa oleifera* Lam. (see notably the articles: OLSON, M.E. 2002, Combining Data from DNA Sequences and Morphology for a Phylogeny of Moringaceae (Brassicales), Systematic Botany 27(1): 55-73; HASSANEIN, A.M.A. AND AL-SOQEE, A.A., 2018, Morphological and genetic diversity of Moringa oleifera and Moringa peregrina genotypes, Horticulture, Environment and Biotechnology 59(2): 251-261). A recent article on *Moringa peregrina* sampled on various locations in Saudi Arabia concluded, by using ITS markers, that there was genetic stability of the species (ALAKLABI, A., 2015, Genetic diversity of Moringa peregrina species in Saudi Arabia with ITS sequences, Saudi Journal of Biological Sciences 22: 186-190) with, however, a high level of intra-population genetic variation.

The species *Moringa peregrina* is found in the rocky environments of Yemen, Oman, Saudi Arabia, Eastern Africa, Sudan, Ethiopia, Eritrea, Somalia and Djibouti. Its presence in Iran appears limited to the south-eastern provinces, but this requires confirmation (PROTA14 = MUNYANZIZA E. AND YONGABI K.A., Vegetable oils/Oleaginous plants, Moringa peregrina (Forssk.) Fiori, http://database.prota.org/protahtml/moringa peregrina_fr.htm, accessed on 10/23/2019). In the Middle East and in Egypt, the species is now only represented by rare dispersed relict stations (with the exception of a few populations at altitude), mainly in the sectors of the Sudan area. *Moringa peregrina* is today also considered as being rare and in danger in Sudan and Yemen. Relative to the other species of its clad, *Moringa peregrina* occupies the most arid and inhospitable habitats. It is apparently more drought-resistant than *Moringa oleifera* which is planted commercially on a large scale in the tropical and subtropical zones. Recent studies have shown that the size and girth of the seeds had a favorable impact on the germination time and the rate and speed of growth of the young individuals (GOMAA N.H. AND PICÓ F.X., 2011, Seed germination, seedling traits, and seed bank of the tree Moringa peregrina (Moringaceae) in a hyper-arid environment, American Journal of Botany 98(6): 1024-1030), indicating an adjustment in the allocation of resources regarding the seed quality rather than the number, which enables *Moringa peregrina* to reproduce efficiently in extreme (hyper-arid) abiotic environments. *Moringa peregrina* seeds have a thicker central mesotesta, in terms of cell layer, than those of *Moringa oleifera.*

A few historical reports exist which tend to indicate that *Moringa peregrina* oil was actively traded at the dawn of Islam in the region of Al-Ula (NASEEF, A.A.S.,1995, *Al-'Ula̅, A study of Cultural and Social Heritage).* The oil produced locally from *Moringa peregrina* is nowadays mainly destined for personal consumption or for local markets. In Saudi Arabia, the leaves were traditionally used as a decoction for internal use for treating diabetes, bowel diseases, ocular diseases and anemias (ABDEL-KADER, M.S., HAZAZI A.M. A., ELMAKKI O.A. AND ALQASOUMI S.I., 2018, A survey on the traditional plants used in Al Kobah village, Saudi Pharmaceutical Journal 26(6): 817-821) and as a diuretic, rubefacient and astringent (AQEEL A.A.M., TARIQ M., MOSSA J.S., AL-YAHYA M.A. AND AL-SAID M.S., 1984, "Plants used in Arabian Folk medicine", Report submitted to Saudi Arabian National Centre for Science and Technology, Riyadh, Saudi Arabia). In Oman, the oil, extracted by women at the end of the summer, is used to combat migraine, fever, burns, lacerations and fractures, constipation and stomach pains, and to combat muscular pain, dryness of the hair and labour pains (GHAZANFAR S.A., 1994, Handbook of Arabian Medicinal Plants, 1st ed., CRC Press, Boca Raton, Ann Arbor, U.S.; GHAZANFAR S.A., 1998, Plants of Economic Importance, cap. 15, in GHAZANFAR, S.A. AND FISHER, M. (ed.) Vegetation of the Arabian Peninsula. Geobotany 25, pages 241-264, Kluwer Academic Publishers, table 11.1, page 247 and 11.7 page 251). It was also used in fragranced compositions (GHAZANFAR S.A., 1998, page 259) and in Oman and Yemen as a face lotion (GHAZANFAR S.A. AND RECHINGER B., 1996, Two multi-purpose seed oils from Oman. Plants for Food and Medicine. Paper presented at the joint meeting of the Society for Economic Botany and International Society for Ethnopharmacology, July 1-7, 1996, London). Traditional uses of Moringa peregrina seed oil is known for treating headaches, fevers, constipation, burns, abdominal pains, back and muscle pains, and childbirth labor pains (BOULOS, L., 2000. Flora of Egypt, vol. I. Al Hadara Publishing, Cairo, p. 238; ELBATRAN, S.A., ABDEL-SALAM, O.M., ABDELSHFEEK, K.A., NAZIF, N.M., ISMAIL, S.I., HAMMOUDA, F.M., 2005. Phytochemical and pharmacological investigation on Moringa peregrina (Forssk) Fiori., Nat. Prod. Sci. 11 (3), 199-206.). The traditional medicinal uses of the seeds include uses for cooking and to cure abdominal pain (VAN DER VOSSEN, H.A.M., MKAMILO, G.S., 2007. PROTA 14: Vegetable Oils. PROTA publishers, Wageningen, pp. 119-120).

Lalas et al. (LALAS, S., GORTZI, O., ATHANASIADIS, V., TSAKNIS, J, AND CHINOU, I., 2012, Determination of Antimicrobial Activity and Resistance to Oxidation of Moringa peregrina Seed Oil, Molecules 17, p. 2330-2334) found that the seed oil of *M. peregrina* was active against S. *aureus, S. epidermidis, Pseudomonas aeruginosa, E. cloacae, K. pneumoniae, E. coli, Candida albicans, C. tropicalis* and *C. glabrata.* The MIC for these respective microbes are 3.5, 3.35, 4.38, 4.8, 4.3, 4.95, 5.7, 3.3, and 3.25 mg/ml.

Tsaknis (TSAKNIS, J., 1998. Characterisation of Moringa peregrina Arabia seed oil. Grasas y Aceites 49 (2), 170-176) has studied the chemical composition of the seed oil *of M. peregrina,* palmitic acid and stearic acid were found to be the major components of the saturated FAs while oleic and gadoleic acids were the predominant unsaturated FAs. Of the sterols, β-sitosterol was the most predominant and among those detected include campesterol, stigmasterol, and brassicasterol. α-, γ-, and δ-tocopherols were also detected.

The study in the fatty acid profiling of the seeds by Osman & Abohassan (2012) also revealed that, of the nine fatty acids identified in the seeds, five were classified as the saturated and the highest amount were oleic acid (53.94% 64.19%), palmitic acid (12.89% 17.88%), and stearic acid (6.63% 11.51%). Another comprehensive analysis of the seed oil has been carried out by Somali et al. (SOMALI, M.A., BAJNEID, M.A., ALFHAIMANI, S.S., 1984. Chemical composition and characteristics of M. peregrina seeds and seed oil. Journal of the American Oil Chemists' Society 61 (1), 85-86) that revealed unsaturated fatty acids to comprise 84.7% of the oil. This unprecedented finding is summarized by the authors as follow: "The *Moringa peregrina kernel contains 1.8% moisture, 54.3% oil, 22.1% protein,* 3.6% *fiber, 15.3% carbohydrate and 2.5% ash. The composition and characteristics of the extracted oil were determined. Gas liquid chromatography of methyl esters of the fatty acids shows the presence of 14.7% saturated fatty acids and 84.7% unsaturated fatty acids. The fatty acid composition is* as *follows: palmitic* 9.3%, *palmitoleic 2.4%, stearic 3.5%, oleic 78.0%, linoleic 0.6%, linolenic 1.6%, arachidonic acid 1.8% and behenic 2.6%.*

Other studies on *M. peregrina* seed oil of Iranian origin (GHARIBZAHEDI, S.M.T., ANSARIFARD, I., HASANABADI, Y., SADEGHI, H., GHAHDERIJANI, M., AND YOUSEFI, R., 2013. Physicochemical properties of Moringa peregrina seeds and its oil. Quality Assurance and Safety Crops and Food 5 (4), 303-309) also revealed similar data: oleic acid (77.9%) was the dominant fatty acid followed by palmitic acid (9.3%), stearic acid (3.5%), and behenic acid (2.6%). All these data and others (e.g., SALAHELDEEN, M., AROUA, M.K., MARIOD, A.A., CHENG, S.F., AND ABDELRAHMAN, M.A., 2014. An evaluation of Moringa peregrina seeds as a source for bio-fuel, Industrial Crops and Products 61, 49-61) suggest that polyunsaturated fatty acids are the main component of M. peregrina seed oil.

The seeds of the Persian *M. peregrina* were studied for their vitamin composition (ASGHARI, G., PALIZBAN, A., AND BAKHSHAEI, B., 2015. Quantitative analysis of the nutritional components in leaves and seeds of the Persian Moringa peregrina (Forssk.) Fiori, Pharmacognosy Research 7 (3), 242-248). Sufficient amounts of vitamin C: 1460.6 mg/100 g/dry weight; and vitamin A: 24.860.7 mg/100 g/dry weight were recorded. Furthermore, elemental analysis in the seeds showed that the calcium content is 1164.8643.4 mg/100 g/dry weight and the potassium content was 572610 mg/100 g/dry weight.

The oil composition of *Moringa peregrina* from Saudi Arabia and Egypt versus *M*. *oleifera* has been studied by Robiansyah et al. 2014 (ROBIANSYAH, I., HAJAR, A.S., AL-KORDY, M.A., AND RAMADAN, A. 2014. Current Status of Economically Important Plant Moringa peregrina (Forrsk.) Fiori in Saudi Arabia: A Review, International Journal of Theoretical and Applied Sciences 6(1), 79-86; table 1, p. 81 & table 2 & 3, p. 82). The oil has higher tocopherols which consist of α- β- and δ-tocopherol, at concentrations of 145, 58 and 66 mg/kg, respectively (TSAKNIS 1998). This high content of tocopherols will serve as protector for the oil during the storage and processing. Other studies showed that *M. peregrina* seed oil could resist up to 10.5 hours at 120°C, which is much higher than of extra virgin olive oil (8.9 hours).

Cosmetic compositions comprising extracts originating from *Moringa oleifera* seeds are known in the cosmetic field. For example, Document US667047 describes oxidatively stable emollients produced from moringa oil or its derivatives. It discloses a composition having high slip characteristic comprising a long-chain oil of *Moringa oleifera,* said long chain oil has a methylene interrupted unsaturation of less than 1%.

Document KR101540333 discloses a mixed extract prepared by purified water extraction on a mixture of *Moringa oleifera, Dictamnus albus* and *Annona muricata* species, extraction is provided at 100 °C for 6 hours and then the extract obtained is filtered. The obtained filtered mixed extract is used for preventing hair loss.

Document KR101947705 discloses a cosmetic composition for preventing hair loss or promoting hair growth, a pharmaceutical composition and a food composition comprising mainly a wood flavour extract and a camellia extract obtained with ethanol extraction, and Moringa oil and black cumin seed oil. The composition comprises 0.01 to 1% by weight of Moringa oil and preferably 0.01%. The *Moringa* species that is described in that document, according to it mechanical lubricant activity, appears to be *Moringa oleifera.*

Finally, document KR102277088 discloses a cosmetic composition comprising a fermented milk protein of camelia oil, an argan oil, and a *Moringa oleifera* oil, and a method for preparing the same, as active ingredients for use in improving scalp or preventing hair loss.

All of the cited prior art discloses composition made of a mixture with different extracts, including extract of the species *M. oleifera,* for preventing or treating hair loss. Given the foregoing, one problem that the invention proposes to solve is that of developing novel products based on a natural extract of the *Moringa peregrina* that may be used in cosmetics or pharmaceutics and that are easy to use for improving capillary density or preventing and treating hair loss.

The Applicant has discovered that a composition comprising, as active ingredient, at least an oily extract of *Moringa peregrina* seeds makes it possible to improve drastically the capillary density of the scalp or skin.

The Applicant has found that this new composition was particularly effective for hair regrowth and/or for slowing down hair loss.

### Summarize

A first aspect of the invention is a cosmetic topical composition for improving capillary density, containing as active ingredient at least an oily extract of *Moringa peregrina* seeds, optionally combined with cosmetically acceptable excipients.

A second aspect of the invention is a method for preparing a composition according to the invention that comprises the following steps, according to which:
a) Shelled seeds of *Moringa peregrina* (Forssk.) Fiori harvested when the fruit is ripe are dried to obtain an internal moisture content of less than 8% and preferentially about 6%,
b) Dried seeds are pressed with a mechanical endless screw press, so as to separate the oil from the rest of the seed in order to obtain, on the one hand, the virgin oil and, on the other hand, a cake,
c) The virgin oil is then decanted naturally, isolated and put in a sterilized container optionally combined with cosmetically acceptable excipients.

A third aspect of the invention is a cosmetic treatment of hair, characterized in that it consists in applying twice a day to the scalp and/or the hair an effective amount of at least about 0.06 g of a composition according to the invention for at least 5 minutes, ideally 10 minutes, and optionally rinsing with water and/or a regular shampoo.

A fourth aspect of the invention is a dermatologic composition for use as a drug, for preventing and treating hair loss, containing as active ingredients at least an oily extract of *Moringa peregrina* seed, optionally with dermatologically acceptable excipients.

The invention will be better understood, and other aims, details, characteristics and advantages thereof will appear more clearly during the following description of several particular embodiments of the invention, given solely by way of illustration and not of limitation.

### Description of the embodiments

In this description, unless otherwise specified, it is understood that when a range is given, it includes the upper and lower limits of said range.

In the present invention, the following definitions apply:
- "topical composition": applying or spreading the active principle according to the invention, or a composition containing said active principle, onto the surface of the skin or the scalp or the hair.
- "cosmetically or dermatologically acceptable": suitable for topical use, in contact with human skin, without any risk of toxicity, incompatibility, instability or allergic response.
- expression "prevention of hair loss" as used herein refers to minimizing the hair loss, preventing hair loss due to various habits and environmental influences such as genetic causes, hormonal imbalance, mental stress of social life, exposure to air pollution, and ingestion of processed foods.
- expression "treatment of hair loss" as used herein refers to minimizing the absence of hair and making hair growth in a region where hair should be normally present but is not due to various illness and treatment that cause the fall of hair.
- "shelled seeds": means that the shell, or pericarp, of the harvested seeds is kept around the seeds.
- "when the fruit is ripe": means that the fruit is ripe, preferentially when the pod is at the start of dehiscence and turns a dark beige to brown color and when a 180° twist of the lower quarter of the pod brings about opening of the valves.
- expression "improving capillary density of hair": means it stops the hair fall, stimulates growth of hair and increases density of hair in that the number of hair par cm2 is higher than that before the cosmetic treatment.
- "about": a margin of plus or minus 10% to 20% relative to the given information (duration, percentage, etc.).
- "active ingredient" or "active agent": sufficient amount of an extract according to the invention to obtain the biological activities described. Depending on the cause of prevention or treatment, the amounts of the active agent may vary in proportions of from 5% to 100% by weight relative to the total weight of the composition.

A first aspect of the invention is a cosmetic topical composition for improving capillary density, containing as active ingredient at least an oily extract of *Moringa peregrina* seeds, optionally combined with cosmetically acceptable excipients.

Thanks to these characteristics, the oily extract of *Moringa peregrina* seeds is proved to be particularly effective for improving capillary density, for hair regrowth and/or for slowing down hair loss.

According to embodiments, such a composition may comprise one or more of the following characteristics.

According to one embodiment, the composition contains as active ingredient the virgin oil of *Moringa peregrina* seeds.

According to a preferred embodiment, the composition contains as active ingredient a winterized oil of *Moringa peregrina* seeds. Such winterized oil is described in following example 1.

According to a preferred embodiment, the seeds are shelled seeds of *Moringa peregrina* (Forssk.) Fiori harvested when the fruit is ripe then dried to obtain an internal moisture content of less than 8% and preferentially about 6%.

According to one embodiment, the composition contains from 5% to 100% by weight of the oily extract of *Moringa peregrina* seeds.

According to a preferred embodiment, the composition contains 100% by weight of the oily extract of *Moringa peregrina* seeds. In that embodiment, the oily extract of *Moringa peregrina* seeds does not contain any excipient or ingredient other than the natural ingredients extracted from the seeds. In a more particularly preferred embodiment, the oily extract is a winterized oil extract.

According to one embodiment, the composition contains between 5% and less than 100% by weight of the oily extract of *Moringa peregrina* seeds and other ingredients for improving the formulation, that is to say for modifying the oily formulation into another formulation more adapted for application.

Since the compositions according to the invention are intended for topical administration, the compositions must thus optionally contain a cosmetically acceptable medium, i.e. a medium that is compatible with the skin and the skin appendages, and cover all cosmetic forms. These compositions may notably be in the form of creams, oil-in-water or water-in-oil emulsions or multiple emulsions, sera, solutions, suspensions, gels, milks, lotions, sticks or even powders, and may be suitable for application to the skin and/or the skin appendages such as hair. These compositions comprise the excipients that are necessary for their formulation, such as solvents, emollients, thickeners, diluents, surfactants, antioxidants, bioactive agents, dyes, preserving agents and fragrances.

The composition according to the invention may in particular consist of a haircare composition, and notably a shampoo, a hair conditioner, a treating lotion, a styling cream or gel, a hair restructuring lotion, a mask, etc. The cosmetic composition according to the invention may notably be used in treatments involving an application which may or may not be followed by rinsing, or alternatively in the form of a shampoo.

The compositions according to the invention may also comprise any additive commonly used in the envisioned field of application and also the adjuvants required for their formulation, such as solvents, thickeners, diluents, antioxidants, dyes, sunscreens, pigments, fillers, preserving agents, fragrances, odor absorbers, cosmetic or pharmaceutical active agents, essential oils, vitamins, essential fatty acids, surfactants, film-forming polymers, etc.

The INCI Dictionary & Handbook ("International Nomenclature of Cosmetic Ingredients" (13th edition, 2010) published by The Personal Care Products Council Inc., Washington, D.C.) describes a wide variety, without limitation, of cosmetic and pharmaceutical ingredients commonly used in the skincare industry, which are suitable for use as additional ingredients in the compositions according to the present invention.

In any case, a person skilled in the art will take care to ensure that these adjuvants and the proportions thereof are chosen such that the desired advantageous properties of the composition according to the invention are not adversely affected.

A second aspect of the invention is a method for preparing a composition according to the invention that comprises the following steps, according to which:
a) Shelled seeds of *Moringa peregrina* (Forssk.) Fiori harvested when the fruit is ripe, the seeds are dried to obtain an internal moisture content of less than 8% and preferentially about 6%,
b) Dried seeds are pressed with a mechanical endless screw press, so as to separate the oil from the rest of the seed in order to obtain, on the one hand, the virgin oil and, on the other hand, a cake,
c) The virgin oil is then isolated and put in a sterilized container optionally combined with cosmetically acceptable excipient.

According to a preferred embodiment, the method for preparing a composition according to the invention comprises the following steps, according to which:
a) Shelled seeds of *Moringa peregrina* (Forssk.) Fiori harvested when the fruit is ripe are dried to obtain an internal moisture content of less than 8% and preferentially about 6%,
b) Dried seeds are pressed with a mechanical endless screw press, so as to separate the oil from the rest of the seed in order to obtain, on the one hand, the virgin oil and, on the other hand, a cake,
c) A first crystallization under moderate stirring is performed on the virgin oil at about 13°C during 60 to 180 minutes, depending on the batch size, followed by vacuum filtration at about 13°C with a filtration cut of 5 µm for obtaining a first winterized oil,
d) A second crystallization of the first filtrate is performed under moderate stirring at about 4°C during 60 to 180 minutes, depending on the batch size, followed by vacuum filtration at about 4°C with a filtration cut of 5 µm for obtaining a second winterized virgin oil,
e) The winterized oil is then isolated and put in a sterilized container optionally combined with cosmetically acceptable excipients.

A third aspect of the invention is a method for cosmetic treatment of hair consisting in applying twice a day to the scalp and/or the hair an effective amount of at least about 0.06 g of a composition according to the invention for at least 5 minutes, ideally 10 minutes, and optionally rinsing with water and/or a regular shampoo.

A fourth aspect of the invention is a dermatologic composition for use as a drug for preventing and treating hair loss containing as active ingredients at least an oily extract of *Moringa peregrina* seed, optionally with dermatologically acceptable excipients.

According to one embodiment, the dermatologic composition is further used as a drug for preventing and alleviating itches.

According to a preferred embodiment, the dermatologic composition contains a virgin oil or winterized oil of *Moringa peregrina* seeds.

According to another preferred embodiment, the seeds are shelled seeds of *Moringa peregrina* (Forssk.) Fiori harvested when the fruit is ripe then dried to obtain an internal moisture content of less than 8% and preferentially about 6%.

According to an embodiment the dermatologic composition contains from 5 to 100% by weight of the oily extract of *Moringa peregrina* seeds.

Although the invention has been described in connection with several particular embodiments, it is obvious that it is in no way limited thereto and that it includes all the technical equivalents of the means described as well as their combinations if these fall within the scope of the invention.

The use of the verb "to comprise", "to contain" or "to include" and of its conjugated forms does not exclude the presence of other elements or other steps than those set out in a claim.

In the claims, any reference sign in parentheses shall not be interpreted as a limitation of the claim.

### Examples

### Example 1: Preparation of a plant extract from Moringa peregrina seed

Shelled seeds of *Moringa peregrina* (Forssk.) Fiori harvested when the fruit is ripe were dried to obtain an internal moisture content of less than 8% and preferentially about 6%, and then pressed with a mechanical endless screw press, so as to separate the oil from the rest of the seed in order to obtain, on the one hand, the virgin oil and, on the other hand, a cake. A first crystallization under moderate stirring is performed on the virgin oil at about 13°C during 60 to 180 minutes (depending on the batch size) followed by vacuum filtration at about 13°C with a filtration cut of 5 µm for obtaining a first winterized oil. A second crystallization of the first filtrate is performed under moderate stirring at about 4°C during 60 to 180 minutes (depending on the batch size) followed by vacuum filtration at about 4°C with a filtration cut of 5 µm for obtaining a second winterized oil. The winterized oil is then isolated and put in a sterilized container. The winterized oil is thereafter called WINTERIZED ALULA PEREGRINA OIL.

### Example 2: Measurement of hair density using Aramo SG^{®} ASG 200F

The use test is conducted at laboratory under dermatologist control. The study was designed to:
- assess the impact of cosmetic on tolerance at the application site as a result of regular application of the WINTERIZED ALULA PEREGRINA OIL, according to the purpose and use of the specified time;
- research leading to confirm or exclude the properties claimed for the cosmetic.

The Evaluation Questionnaire form, the characteristics of the panel and number of subjects were in line with the requirements. The WINTERIZED ALULA PEREGRINA OIL was applied by the subjects in accordance with the declared method of use.

Declarations are considered confirmed only if the ratio of the received positive responses to the total number of subjects participating in the study is above 50%.

Description in Table 1 of the tested subjects.

**[Table 1]**

| | | |
|---|---|---|
| **GENERAL** | Sign an informed consent to participate in the study, were informed about the purpose of the study, the manner of its conduct and the possible side effects. | |
| | The site of product application without irritation and changes requiring pharmacological treatment (specialist evaluation) | |
| | Amount of subjects: | 21 |
| | Gender: | Women, Men |
| **SPECIFIC** | Age: | 43 - 72 |
| | Hair and scalp type: | All |
| | Others: | Adults with itchy scalp, significant or daily hair loss, dandruff |

The qualified subjects received the tested WINTERIZED ALULA PEREGRINA OIL, specially developed questionnaire and were obliged to:
- use of the products according to the method of use,
- during the test any other products of similar effects must not be used,
- a detailed evaluation of the tested products by using the received questionnaire,
- in case of any side effects on skin they should immediately stop using the products and consult specialist

The test results may be affected by such factors as:
- type and condition at the site of product application,
- inter-individual genetic characteristics,
- individual preferences of subjects.

The aim of the study is to assess the direct impact of the WINTERIZED ALULA PEREGRINA OIL of the invention according to example 1 on hair density. The test has been conducted using special measuring device manufactured by ARAM HUVIS Co., Ltd. - ARAMO SG^{®} ASG 200F. Instrumental study has been carried out on 21 subjects. The measurements in zoom 60-times has been performed at the specified zone - before product application (D0), after 56 (D56) and 84 days (D84) of regular use. Every day, each subject had to apply twice, morning and evening, 3 drops of the WINTERIZED ALULA PEREGRINA OIL (one drop is about 0.02 g).

The study has been carried out in an air-conditioned room in the temp. of 20±2°C and relative humidity 50±10%. Table 2 hereunder gives the mean results of hair density at the site of the products application on measurements before application (D0), after 56 (D56) and 84 days (D84) of regular use in [average amount of hair/cm²].

**[Table 2]**

| Subject's no. | Before (L0) | After 56 days (D56) | After 84 days (D84) | Difference (D56-D0) | Difference (D84-D0) |
|---|---|---|---|---|---|
| 1. | 138 | 143 | 144 | 5 | 7 |
| 2. | 127 | 138 | 141 | 11 | 14 |
| 3. | 117 | 123 | 134 | 7 | 18 |
| 4. | 143 | 149 | 152 | 7 | 9 |
| 5. | 138 | 141 | 144 | 3 | 7 |
| 6. | 113 | 122 | 134 | 8 | 21 |
| 7. | 138 | 144 | 155 | 7 | 17 |
| 8. | 138 | 152 | 158 | 14 | 21 |
| 9. | 111 | 127 | 131 | 16 | 20 |
| 10. | 157 | 172 | 178 | 16 | 22 |
| *(11.)** | *(150)** | *(Untraceable)** | | | |
| 12. | 172 | 178 | 194 | 6 | 22 |
| 13. | 97 | 118 | 127 | 21 | 30 |
| 14. | 111 | *(Untraceable)** | 132 | *(Untraceable)** | 21 |
| 15. | 143 | 159 | 164 | 16 | 21 |
| 16. | 160 | 172 | 178 | 12 | 18 |
| 17. | 92 | 118 | 127 | 26 | 35 |
| 18. | 97 | 115 | 128 | 19 | 32 |
| 19. | 128 | 144 | 152 | 16 | 24 |
| 20. | 96 | 118 | 127 | 23 | 31 |
| 21. | 102 | 120 | 125 | 19 | 24 |
| Mean | 125,7 | 139,7 | 146,3 | 13,2 | 20,6 |
| Min | 92,3 | 115,0 | 125,0 | 3,3 | 6,7 |
| Max | 172,0 | 178,0 | 194,0 | 26,0 | 34,7 |
| SD | 23,4 | 20,2 | 19,9 | 6,6 | 7,7 |
| Median | 127,5 | 141,0 | 142,7 | 14,0 | 21,0 |
| Δ% | | | | 10% | 16% |
| % of subjects with the positive effect | | | | 100% | 100% |

Conclusion: the composition comprising the WINTERIZED ALULA PEREGRINA OIL (winterized oil) improves hair density after 56 days of regular use of about 10% (average) and after 84 days of regular use of about 16% (average).

### Example 3: Assessment of the composition's impact on tolerance at the site of application

On the basis of medical examinations and interviews collected from subjects, it was found that the tested composition according to example 1 (WINTERIZED ALULA PEREGRINA OIL) was very well tolerated at the application site. In all subjects who finished the study, during the regular application, in the interviews there was no negative symptom and feeling that might indicate an intolerance to any component of the product, such as irritation, burning sensation, redness or itching. The product did not cause dryness at the site of application in any subject.

Based on the results of use test - properties of composition WINTERIZED ALULA PEREGRINA OIL were assessed as follows in Table 3.

**[Table 3]**

| | | |
|---|---|---|
| The hair is softer after application. | 70% | positive responses |
| The product gives a feeling of protection to the hair. | 75% | positive responses |
| The hair is silkier after application. | 80% | positive responses |
| The product removes bad hair odor. | 55% | positive responses |
| It leaves a neutral odor or even a pleasant odor on hair. | 75% | positive responses |
| The product gives the feeling of a cleansed scalp. | 75% | positive responses |
| The product soothes itching. | 70% | positive responses |
| The hair looks stronger after application. | 85% | positive responses |
| The hair looks longer after application. | 55% | positive responses |
| The product provides an improvement in itching and feeling of well-being. | 65% | positive responses |
| The product volumizes the hair. | 70% | positive responses |
| The product does not cause the feeling of dryness of the hair after application. | 85% | positive responses |
| The product improves the texture of my hair. | 75% | positive responses |
| The hair looks nourished after application. | 85% | positive responses |
| There is not a change in the color of the hair. | 70% | positive responses |
| The product redone shine to the hair. | 80% | positive responses |
| The product noticeably reduces the symptoms of dandruff. (6 subjects = 100%) | 50% | positive responses |
| I have not encountered any difficulties in handling and the use of the product. | 65% | positive responses |
| I like cosmetic quality of the product. | 75% | positive responses |

Conclusion: the results obtained in the test allow to conclude that the composition according to the invention, used as intended, is very well tolerated by the people tested in whom there is not a contraindication of its use.

It can as well be concluded that the composition helps alleviating itches since for 70% of tested people, the product soothes itches.

Under the study conditions, after 84 days of regular application it is concluded that the composition WINTERIZED ALULA PEREGRINA OIL:
- was very well tolerated at the application site,
- properties declared have been confirmed based on a subjective questionnaire:
   ï The hair is softer after application.
   ï The product gives a feeling of protection to the hair.
   ï The hair is silkier afterapplication.
   ï The product removes bad hair odor.
   ï It leaves a neutral odor or even a pleasant odor on hair.
   ï The product gives the feeling of a cleansed scalp.
   ï The product soothes itching.
   ï The hair looks stronger after application.
   ï The hair looks longer after application.
   ï The product provides an improvement in itching and feeling of well-being.
   ï The product volumizes the hair.
   ï The product does not cause the feeling of dryness of the hair after application.
   ï The product improves the texture of myhair.
   ï The hair looks nourished afterapplication.
   ï There is not a change in the color of the hair.
   ï The product redone shine to the hair.
   ï I have not encountered any difficulties in handling and the use of the product.
   ï I like cosmetic quality of the product.

### Example 4: comparative data of the fatty acid profile for Moringa oleifera and Moringa peregrina seed oil

**[Table 4]**

| | | *Moringa oleifera* KSA FDA-Saso^{∗∗∗} | *Oleifera* average seed oil | Data for *Moringa peregrina* virgin seed oil | Data for one winterized seed oil batch according to the invention |
|---|---|---|---|---|---|
| Lauric acid | | | | | |
| Myristic acid | C14:0 | 0.00-0.20 | 0,1 | 0.1 | 0.1 |
| Palmitic acid | C16:0 | 5.90-7.00 | 6,45 | 9.6 | 8.2 |
| Palmitoleic acid | C16:1 | 0.90-2.90 | 1,9 | 2.3 | 1.8 |
| Margaritic acid | C17:0 | .. | .. | 0.1 | - |
| Stearic acid | C18:0 | 4.33-6.50 | 5,4 | 5.2 | 3.6 |
| Oleic acid | C18:1 | 67.00-76.00 | 71,5 | 72.5 | 80.3 |
| Linoleic acid | C18:2 | 0.19-1.96 | 1,1 | 0.6 | 0.5 |
| Linolenic acid | C18:3 | 0.00-2.50 | 1,25 | -- | -- |
| Arachidic acid | C20:0 | 2.82-4.20 | 3,51 | 2.9 | 1.8 |
| Gadoleic (Gondoic, 11-eicosenoic)) acid | C20:1 | 1.20-2.64 | 1,92 | 2.2 | 2.3 |
| Behenic acid | C22:0 | 5.00-8.00 | 6,5 | 3.7 | 1.0 |
| Lignoceric acid | C24:0 | | | 0.8 | 0.4 |
| Cerotic acid | C26:0 | 0.00-1.21 | 0,61 | -- | -- |
| ^{∗∗∗} range used in KSA by FDA as reference for oil | | | | | |
| .. A fatty acid not detected | | | | | |

### Example 5: Hair styling « Waxy Jelly » Hair Booster: wet look hair styling product

**[Table 5]**

| **Commercial Name** | **%** | **INCI** | **% Active** | **% Actuals** |
|---|---|---|---|---|
| Water | QS | *Aqua* | 100 | *60,45* |
| Sodium alginate | 1,5 | *Algin* | 100 | 1,5 |
| Oligogeline | 2 | *Aqua* & *Chondrus Crispus Extract* | 5 ; 95 | *0,1 ; 1,9* |
| WINTERIZED ALULA PEREGRINA OIL | 6 | *Moringa peregrina* seed extract | 100 | 6 |
| Cetearyl alcohol | 2 | *Cetearyl alcohol* | 50 ; 50 | *1* ; *1* |
| Vitamin E | *0,2* | *Tocopheryl acetate* | 100 | *0,2* |
| Luviskol VA 64 W | 14 | *VP*/*VA Copolymer* | 100 | 14 |
| Alcohol 95% | 12 | *Alcohol* & *Aqua* | 95 ; 5 | 11,4 ; *0,6* |
| Citric acid | *0,1* | *Citric acid* | 100 | *0,1* |
| Benzyl alcohol MLW-00254 | 1 | *Benzyl alcohol* | 100 | 1 |
| Aminat G | 1 | *Glycerin* & *Ethyl Lauroyl Arginate HCL* | 50 ; 50 | *0,5; 0,5* |

### Example 6: Stimulating Shampoo Hair Booster: Apply in a thick layer and let stand from 5 to 10 min. To lather and to rinse with clear water

**[Table 6]**

| **Commercial Name** | **%** | **INCI** | **% Active** | **% Actuals** |
|---|---|---|---|---|
| Water | QS | Aqua | 100 | 72,7 |
| Aminat G | 1 | *Glycerin* & *Ethyl Lauroyl Arginate HCL* | 50 ; 50 | 0,5 ; 0,5 |
| Frametime CXG | 4,5 | Bentonite & Xanthan gum & Sodium stearoyl glutamate & Citric acid | 84 ; 6 ; 5 ; 5 | 3,78 ; 0,27 ; 0,225 ; 0,225 |
| Elfan AT 84 | 4 | Sodium cocoyl isethionate | 100 | 4 |
| Sulfetal C90C | 5 | Sodium coco-sulfate | 100 | 5 |
| WINTERIZED ALULA PEREGRINA OIL | 10 | *Moringa peregrina* seed extract | 100 | 10 |
| DUB MCT 5545 | 2 | Caprylic / Capric triglyceride | 100 | 2 |

### Example 7: Hair loss Cream: Apply morning and evening on scalp and massage in. Brush for hair styling. Light wet look if applied to lengths.

**[Table 7]**

| **Commercial Name** | **%** | **INCI** | **% Active** | **% Actuals** |
|---|---|---|---|---|
| Water | QS | Aqua | 100 | 72,5 |
| Aminat G | *1* | *Glycerin* & *Ethyl Lauroyl Arginate HCL* | 50 ; 50 | 0,5 ; 0,5 |
| Frametime CXG | 5 | Bentonite & Xanthan gum & Sodium stearoyl glutamate & Citric acid | 84 ; 6 ; 5 ; 5 | 4,2 ; 0,3 ; 0,25 ; 0,25 |
| Xanthan gum FF | 0,2 | Xanthan gum | 100 | 0,2 |
| Cetearyl stearyl alcohol 50/50 | 1,5 | Cetearyl alcohol | 100 | 1,5 |
| WINTERIZED ALULA PEREGRINA OIL | 19 | *Moringa peregrina* oil extract | 100 | 18,5 |

### Example 8: Hair Growth Mask: Apply in a thick layer on scalp and let stand from 5 to 10 min. To rinse before washing with shampoo. To rinse with clear water.

**[Table 8]**

| **Commercial Name** | **%** | **INCI** | **% Active** | **% Actuals** |
|---|---|---|---|---|
| Water | QS | Aqua | 100 | 58 |
| Unipure white LC987 EM | 2 | Cl 77891 &Silica | 98 ; 2 | 1,96 ; 0,04 |
| White clay | 18 | Kaolin | 100 | |
| Frametime CX | 6 | Bentonite&Xanthan gum&Citric acid | 90 ; 5 ; 5 | 5,4 ; 0,3 ; 0,3 |
| Sericite GMS-4C | 1 | Mica | 100 | 1 |
| Propanediol - Massocare | 5 | Propanediol | 100 | 5 |
| Vegetable glycerin - Palmera G995E | 7 | Glycerin | 100 | 7 |
| WINTERIZED ALULA PEREGRINA OIL | 20 | *Moringa peregrina* seed extract | 100 | 20 |
| Aminat G | 1 | *Glycerin* & *Ethyl Lauroyl Arginate HCL* | 50 ; 50 | 0,5 ; 0,5 |

### Example 9: Hair Booster Dry Oil: Haircare product. Apply morning and evening on scalp and massage in. Brush for hair styling. Light wet look if applied to lengths.

**[Table 9]**

| Commercial Name | % | INCI | % Active | % Actuals |
|---|---|---|---|---|
| WINTERIZED ALULA PEREGRINA OIL | 50 | *Moringa peregrina* seed axtract | 100 | 50 |
| Ephyster MCR | | *Brassica napus* extract | 100 | 49 |
| Vitamin C Tetra E | 1 | Ascorbyl tetraisopalmitate | 100 | 1 |

### Example 10: Conditioner Hair Booster Powder

| **Commercial Name** | **%** | **INCI** | **% Active** | **% Actuals** |
|---|---|---|---|---|
| Frametime CXG | 52 | Bentonite&Xanthan gum&Sodium stearoyl glutamate&Citric acid | 84; 6; 5 ; 5 | 43,68 ; 3,12 ; 2,6 ; 2,6 |
| Xanthan gum FFPC | 3,9 | Xanthan gum | 100 | 3,9 |
| Maltodextrin | 6 | Maltodextrin | 100 | 6 |
| Chitoveg (chitosan plant origin) | 4,1 | Chitosan | 100 | 4,1 |
| Rice starch | 6,3 | Ozyza sativa starch | 100 | 6,3 |
| Monohydrate citric acid F6000 | 3 | Citric acid | 100 | 3 |
| Cosmegreen ES1822+ | 4,9 | Arachidyl/Behenyl Betainate Esylate&Arachidyl/Behenyl Alcohol | 100 | 4,9 |
| WINTERIZED ALULA PEREGRINA OIL | 19 | *Moringa peregrina* seed extract | 100 | 19 |
| NS EX-81 | 2 | Polyglyceryl-8 oleate | 100 | 2 |

To use after shampoo. To apply on scalp and let stand from 5 to 10 min:
- To sprinkle on wet hair and massage in scalp
- To let stand in thick layers up to 10min
- To rinse with clear water

## Claims

1. A cosmetic topical composition for improving capillary density, containing as active ingredient at least an oily extract of *Moringa peregrina* seeds, optionally combined with cosmetically acceptable excipients.

2. Composition according to claim 1 **characterized in that** the oily extract is the virgin oil of *Moringa peregrina* seeds.

3. Composition according to claim 1 **characterized in that** the oily extract is a winterized oil of *Moringa peregrina* seeds.

4. Composition according to any one of claims 1 to 3 **characterized in that** the seeds are shelled seeds of *Moringa peregrina* (Forssk.) Fiori harvested when the fruit is ripe then dried to obtain an internal moisture content of less than 8% and preferentially about 6%.

5. Composition according to any one of claims 1 to 4, **characterized in that** it contains from 5% to 100% by weight of the oily extract of *Moringa peregrina* seeds.

6. Composition according to claim 5, **characterized in that** it contains 100% by weight of the oily extract of *Moringa peregrina* seeds.

7. Composition according to claim 5 **characterized in that** it contains between 5% and less than 100% by weight of the oily extract of *Moringa peregrina* seeds and other ingredients for improving the formulation.

8. A method for preparing a composition according to claims 1 and 2, **characterized in that** it comprises the following steps, according to which:
a) Shelled seeds of *Moringa peregrina* (Forssk.) Fiori harvested when the fruit is ripe are dried to obtain an internal moisture content of less than 8% and preferentially about 6%,
b) dried seeds are pressed with a mechanical endless screw press, so as to separate the oil from the rest of the seed in order to obtain, on the one hand, the virgin oil and, on the other hand, a cake,
c) The virgin oil is then isolated and put in a sterilized container optionally combined with cosmetically acceptable excipient.

9. A method for preparing a composition according to claim 3, **characterized in that** it comprises the following steps, according to which:
a) Shelled seeds of *Moringa peregrina* (Forssk.) Fiori harvested when the fruit is ripe are dried to obtain an internal moisture content of less than 8% and preferentially about 6%,
b) dried seeds are pressed with a mechanical endless screw press, so as to separate the oil from the rest of the seed in order to obtain, on the one hand, the virgin oil and, on the other hand, a cake,
c) A first crystallization under moderate stirring is performed on the virgin oil at about 13°C during 60 to 180 minutes depending on the batch size, followed by vacuum filtration at about 13°C with a filtration cut of 5 µm for obtaining a first winterized oil,
d) A second crystallization of the first filtrate is performed under moderate stirring at about 4°C during 60 to 180 minutes depending on the batch size followed by vacuum filtration at about 4°C with a filtration cut of 5 µm for obtaining a second winterized oil,
e) The winterized oil is then isolated and put in a sterilized container optionally combined with cosmetically acceptable excipients.

10. A method for cosmetic treatment of hair, **characterized in that** it consists in applying twice a day to the scalp and/or the hair an effective amount of at least about 0.06 g of a composition according to any one of claims 1 to 7 for at least 5 minutes, ideally 10 minutes, and optionally rinsing with water and/or a regular shampoo.

11. Dermatologic composition for use as a drug for preventing and treating hair loss containing as active ingredients at least an oily extract of Moringa peregrina seed, optionally with dermatologically acceptable excipients.

12. Dermatologic composition according to claim 11 for further use as a drug for preventing and alleviating itches.

13. Dermatologic composition according to claims 11 and 12 **characterized in that** the oily extract is a virgin oil or a winterized oil of *Moringa peregrina* seeds.

14. Dermatologic composition according to any of claims 11 to 13 **characterized in that** the seeds are shelled seeds of *Moringa peregrina* (Forssk.) Fiori harvested when the fruit is ripe then dried to obtain an internal moisture content of less than 8% and preferentially about 6%.

15. Dermatologic composition according to claim 13, **characterized in that** it contains from 5 to 100% by weight of the oily extract of *Moringa peregrina* seeds.
